# EUROPÄISCHE PATENTSCHRIFT

(11) **EP 3 441 616 B1**
(45) Veröffentlichungstag und Bekanntmachung des Hinweises auf die Patenterteilung: **19.04.2023**
(21) Anmeldenummer: 18195089.0
(22) Anmeldetag: 22.09.2009
(51) Int. Cl.: F04D 3/00, F04D 29/18, F04D 29/24

(54) **KOMPRIMIERBARER ROTOR FÜR EINE FLUIDPUMPE**
COMPRESSIBLE ROTOR FOR A FLUID PUMP
ROTOR POUVANT ÊTRE COMPRIMÉ POUR UNE POMPE À FLUIDE

(43) Veröffentlichungstag der Anmeldung: 13.02.2019
(62) Teilanmeldung aus: 09075438.3
(73) Patentinhaber: ECP Entwicklungsgesellschaft mbH, 52074 Aachen (DE)
(72) Erfinder: Schumacher, Jörg, 14513 Teltow (DE); Scheckel, Mario, 14195 Berlin (DE); Schlicht, Henning, 14542 Werder (DE)
(74) Vertreter: Pfenning, Meinig & Partner mbB

(56) Entgegenhaltungen:
- WO-A2-2007/112033
- US-A1- 2003 135 086
- US-A1- 2009 062 597

## Beschreibung

Die vorliegende Erfindung liegt auf dem Gebiet des Maschinenbaus, insbesondere der Feinwerktechnik, und bezieht sich auf Rotoren für Fluidpumpen.

Rotationspumpen sind hinlänglich bekannt, jedoch werden diese ständig, insbesondere für Spezialanwendungen, verbessert. So sind Axialpumpen bekannt geworden, die einen in axialer Richtung Fluid fördernden Rotor in einem Gehäuse aufweisen, wobei Rotor und Gehäuse verformbar, vorteilhaft komprimierbar sind, um diese vor dem Betrieb an einen gewünschten, schwierig zugänglichen Einsatzort zu bringen und sie dort zu dekomprimieren und zu betreiben.

Solche Pumpen werden in Mikrobauform beispielsweise in der Medizin eingesetzt, um in den Körper eines Patienten, beispielsweise über eine Blutbahn, eingebracht zu werden und dort, entweder im Blutgefäß oder in einer Herzkammer, betrieben zu werden.

Die Pumpen können derart komprimiert werden, dass sie durch das Blutgefäß geschoben und dann gegebenenfalls in einem größeren Körperhohlraum dekomprimiert werden können, um den Rotor zur Entfaltung zu bringen und eine hohe Förderleistung zu erzielen.

Ein komprimierbarer Rotor ist beispielsweise aus der US 6 860 713 bekannt.

Ein anderer Rotor ist aus der US 7 393 181 B2 bekannt. Die bekannten Lösungen basieren entweder auf der Elastizität und Verformbarkeit des Materials des Rotors oder auf mechanischen Konstruktionen wie dem Vorsehen von Knickstellen oder Gelenken zur Faltung und Entfaltung der einzelnen Bauteile.

Solche Konstruktionen haben oft den Nachteil, dass die Komprimierbarkeit begrenzt ist, da beispielsweise die Nabe eines Rotors unverändert bleibt, dass aufwendige Gelenke vorgesehen werden müssen, die im Betrieb stabilisiert werden, und dass teilweise superelastische Werkstoffe verwendet werden, wie Gedächtnislegierungen, die abhängig von der Umgebungstemperatur ihre Form ändern.

Diese Konstruktionen machen oft den Einsatz von Verbundwerkstoffen nötig, und es ist schwierig, beim Aufbau von Stützkonstruktionen den Fluss des zu fördernden Fluids nicht zu behindern und gegebenenfalls auch die Schädigung des Fluids weitestgehend auszuschließen. Dies ist insbesondere bei der Förderung von Blut wichtig, das hochfunktionale und auch mechanisch anfällige Bestandteile enthält. Ferner betrifft die Druckschrift US 2003/135086 A1 ein aufblasbares Zirkulationsassitenzgerät verwandter Art.

Der vorliegenden Erfindung liegt vor dem Hintergrund des Standes der Technik die Aufgabe zugrunde, in möglichst einfacher Weise einen Rotor zu schaffen, der konstruktiv einfach aufgebaut, in hohem Maße reversibel komprimierbar und im Betrieb zuverlässig ist.

Die Aufgabe wird gemäß der Erfindung mit den Merkmalen des Patentanspruchs 1 gelöst.

Der erfindungsgemäße Rotor für eine Fluidpumpe weist wenigstens ein Schaufelblatt und wenigstens einen verformbaren, mit einem Fluid gefüllten oder füllbaren Hohlraum auf.

Dadurch ergibt sich eine Volumenkompressibilität des Rotors, die an sich bei einer Kompression schon zu einer Verringerung des Rotorvolumens und gegebenenfalls des Rotordurchmessers führt. Zusätzlich können die verschiedenen Bauteile des Rotors, wie beispielsweise Schaufelblätter, noch gebogen und in Richtung der Rotorachse gedrückt werden, um den Durchmesser weiter zu verringern.

Der Rotor zeichnet sich damit durch eine Materialmischung oder ein Material aus, das durch Kompression von einer ersten, geringeren Dichte bzw. einem ersten geringeren spezifischen Gewicht auf eine zweite, höhere Dichte oder ein höheres spezifisches Gewicht überführbar ist. Dabei können die Hohlräume geschlossen und mit einem Gas wie beispielsweise Luft oder Stickstoff oder einem Edelgas bzw. einem anderen bioinerten Gas gefüllt sein, das durch Druck leicht volumenkomprimierbar ist.

Derartige geschlossene Hohlräume tendieren dazu, sich bei Wegfall einer äußeren Druckkraft durch die Gaselastizität wieder auszudehnen, so dass der Rotor sich, sobald er an den Einsatzort gebracht ist, selbsttätig wieder entfalten kann. Zumindest aber wird die Entfaltungsbewegung durch die Gaselastizität unterstützt.

Es können zudem allerdings auch Gasleitungen zu dem Rotor vorgesehen sein, die in einem oder mehreren Hohlräumen enden und das Aufpumpen der Hohlräume aktiv erlauben. Über die gleichen Leitungen kann gegebenenfalls auch das Gas zur Komprimierung abgesaugt werden.

Ebenso kann mit einer Flüssigkeit verfahren werden, wenn diese in die Hohlräume eingeführt wird. Befindet sich eine Flüssigkeit in den Hohlräumen, so ist diese üblicherweise sehr viel weniger komprimierbar, jedoch kann sie durch die geeignete Wahl der Viskosität im Zusammenspiel mit den übrigen konstruktiven Teilen des Rotors eine hohe Beweglichkeit und damit Komprimierbarkeit ermöglichen und dennoch im Betrieb durch die Inkompressibilität nach Entfaltung des Rotors eine gewisse Rotorstabilität unterstützen.

Die Hohlräume können auch offen gestaltet werden, wobei damit ebenfalls eine hohe Kompressibilität gegeben ist. Das Material, das die Hohlräume begrenzt, muss dann entsprechend elastisch ausgebildet sein. Dies kann zum Beispiel bei einem offenporigen Schaumstoff gegeben sein.

Die Erfindung kann auch vorteilhaft dadurch ausgeführt werden, dass der/die Hohlraum/Hohlräume wenigstens teilweise von einer teildurchlässigen Membran begrenzt ist/sind.

In diesem Fall kann ein Hohlraum mit einer Flüssigkeit gefüllt sein, die zusammen mit der eingesetzten Membran und in Abhängigkeit von der Flüssigkeit, in der die Pumpe einsetzbar ist, insbesondere menschlichem Blut, aufgrund von Osmose eine Diffusion in den Hohlraum hinein zulässt, die zu einer Druckerhöhung und zu einem Aufpumpen des Rotors führt.

Ebenso können auch Stoffe eingesetzt werden, die nach dem Inkontakttreten mit der zu fördernden Flüssigkeit zu Schwellprozessen infolge von Aufsaugen der Flüssigkeit führen und somit über eine Volumenzunahme eine Dekomprimierung des Rotors unterstützen.

Im Falle des Osmoseprozesses bietet sich das Füllen der Hohlräume mit einem Salz oder einer Salzlösung an, deren Salzkonzentration höher ist als die der zu fördernden Flüssigkeiten.

Vorteilhaft kann auch vorgesehen sein, dass zumindest der überwiegende Teil der Hohlräume von festem Material des Rotors umgeben und über Öffnungen mit dem Außenbereich und/oder untereinander verbunden ist/sind. In diesem Fall kann bei der Kompression ein Fluidtransport über die Hohlräume und gegebenenfalls auch aus dem Rotor heraus geschehen, so dass die entsprechenden Hohlräume leicht vollständig komprimierbar sind.

Der Rotor kann beispielsweise teilweise aus einem porösen Material wie Schaumstoff, insbesondere Polyurethan, bestehen. Ein solcher Schaumstoff kann offen- oder geschlossenporig sein. Im Falle eines offenporigen Schaumstoffs beruht die Elastizität auf dem stützenden, die Poren umgebenden Material, das sich nach einer Komprimierung von selbst wieder in seine ursprüngliche Form bewegt, wobei das Gas oder Fluid in die Poren zurückströmen kann. Durch die begrenzten Strömungsquerschnitte der Verbindungen der Hohlräume/Poren untereinander ist eine Zeitkonstante bei der Komprimierung/Dekomprimierung in gewissen Grenzen wählbar. Diese kann dafür sorgen, dass im Betrieb der Pumpe schlagartigen Deformationen des Rotors durch ungleichmäßige mechanische Belastung entgegengewirkt wird.

Erfindungsgemäß ist vorgesehen, dass der Rotor wenigstens einen Hohlraum aufweist, der in einer ersten Richtung eine größere Ausdehnung aufweist als in den im Wesentlichen senkrecht dazu stehenden Richtungen.

Das Vorsehen derartiger anisotroper Hohlräume erlaubt bei richtiger Positionierung auch die Verwirklichung anisotroper mechanischer Eigenschaften des Rotors. Hierdurch ist es möglich, diesen radial einfach und mit geringem Kraftaufwand komprimierbar zu gestalten, ohne dass dieselbe leichte Verformbarkeit im Betrieb durch den dynamischen Widerstand des zu fördernden Fluids eintritt.

Im Betrieb ist damit der Rotor gegenüber den axial und in Umfangsrichtung wirkenden Kräften stabilisiert, während er einer radialen Komprimierung relativ geringe Widerstände entgegensetzt.

Die entsprechenden Hohlräume können im Querschnitt beispielsweise rund, sechseckig, dreieckig oder viereckig ausgebildet sein und beispielsweise Strangform aufweisen, so dass ihr Querschnitt entlang ihrer Länge überall im Wesentlichen gleich ist. Hierdurch ergibt sich eine Symmetrie, die der Stabilität des Rotors dient.

Entsprechende Hohlräume können beispielsweise besonders vorteilhaft in wenigstens einem Schaufelblatt vorgesehen sein, da diese einerseits den größten Anteil an der Durchmesserreduktion des Rotors tragen und andererseits den höchsten dynamischen Kräften im Betrieb ausgesetzt sind.

Dennoch kann das Schaufelblatt derart stabil ausgeführt sein, dass es selbsttragend ist und dass sogar auf eine Nabe verzichtet werden kann. Ein solches Schaufelblatt kann beispielsweise als ein wendelförmig um eine Achse gebogener flacher Körper, insbesondere aus Schaumstoff, ausgebildet sein. Beispielsweise kann zu seiner Herstellung eine Polyurethan-Schaumstoffplatte wunschgemäß in ebener Form zugeschnitten, danach um eine Achse verdreht und dann gehärtet oder versteift werden. Die Form des Schaufelblattes ist damit stabilisiert, jedoch eine elastische Komprimierbarkeit weiterhin gegeben.

Es kann jedoch auch vorgesehen sein, ein derartiges Schaufelblatt oder einen gesamten Rotor durch Spritzen eines Schaumstoffs in eine vorgefertigte Form zu erzeugen.

Die Erfindung kann auch bei mit Naben versehenen Rotoren eingesetzt werden, und in diesem Fall kann insbesondere der Nabenkörper die erfindungsgemäßen Hohlräume aufweisen bzw. wenigstens teilweise aus einem Schaumstoff hergestellt sein.

Werden anisotrope Hohlräume in dem Rotor vorgesehen, so ist es vorteilhaft, diese mit der Richtung ihrer größten Stabilität entlang der Kraft/Spannungsverlaufslinien, die sich im Betrieb innerhalb des Rotors einstellen, ausgerichtet werden.

Beispielsweise können die Längsachsen von strangförmigen Hohlkörpern, wie beispielsweise Wabenkörpern, senkrecht zur Schaufelblattoberfläche ausgerichtet sein, um die in dieser Richtung bzw. Umfangsrichtung wirkenden Kräfte abzufangen.

Die Aufgabe, einen möglichst einfach aufgebauten Rotor zu schaffen, der in hohem Maße (insbesondere reversibel) komprimierbar ist und im Betrieb zuverlässig ist, wird außerdem durch einen komprimierbaren Rotor für eine Fluidpumpe mit wenigstens einem Schaufelblatt gelöst, wobei der Rotor so aufgebaut ist, dass er einen komprimierten und einen dekomprimierten Zustand einnehmen kann und die mittlere Dichteänderung des Rotormaterials zwischen komprimiertem und dekomprimiertem Zustand mindestens 10 % beträgt.

Wichtig in Abgrenzung zum Stand der Technik ist hierbei, dass die Volumenänderung vor allem auch durch Änderungen der Dichte des Rotormaterials zustande kommt. Es handelt sich hier also nicht um bloße elastische Verformungsvorgänge, bei denen die mittlere Dichte des Rotormaterials im Wesentlichen konstant ist. Die genannte Dichteänderung ist "temperaturbereinigt", d. h., es wird die Dichteänderung bei beispielsweise 36°C warmem Rotor zugrunde gelegt für den komprimierten sowie dekomprimierten Zustand.

Zur Herstellung dieser Dichteänderung können die in dieser Anmeldung genannten Ansätze gewählt werden.

Hierbei kommen sowohl reversible als auch irreversible Verfahren in Betracht. Dies sind z. B. osmotische Verfahren, allerdings auch Verfahren, bei denen offenporiger oder geschlossenporiger Schaumstoff Verwendung findet.

Die Dichteänderung des Rotors muss nicht an allen Stellen gleichmäßig sein, beispielsweise ist es möglich, dass im Bereich einer Nabe bzw. des Schaufelblattes wegen der dort eventuell gewünschten höheren Steifigkeit eine kleinere Dichteänderung erzielt wird und im Bereich eines virtuellen "Gelenkes" zwischen Nabe und Schaufelblatt eine stärkere Kompression stattfindet.

Die mittlere Dichteänderung des Gesamtrotors kann bevorzugt auch noch stärker sein, beispielsweise mindestens 15 %, alternativ mindestens 20 % betragen. Bezogen auf einen Kunststoff sind beispielhafte Ausgangsgrößen für die Dichte 0,01...2 g/cm³ im dekomprimierten Zustand und 0,05...3 g/cm³ im komprimierten Zustand.

Die Erfindung bezieht sich außer auf einen Rotor der beschriebenen Art auch auf eine Fluidpumpe mit einem derartigen Rotor, bei der ein komprimierbares, den Rotor umgebendes Gehäuse vorgesehen ist.

Erfindungsgemäß kann auch das Gehäuse wenigstens teilweise aus einem Material bestehen, das Hohlräume aufweist, insbesondere aus einem Schaumstoff, beispielsweise Polyurethan. Auf diese Weise kann das Gehäuse gemeinsam mit dem Rotor einfach komprimiert und dekomprimiert werden.

Im Folgenden wird die Erfindung anhand eines Ausführungsbeispiels in einer Zeichnung gezeigt und anschließend beschrieben.

Dabei zeigt
- Fig. 1: eine schematische Ansicht einer Axialpumpe im Einsatz im Körper eines Patienten,
- Fig. 2: eine vergrößerte Ansicht einer Pumpe, teilweise im Längsschnitt,
- Fig. 3: einen Rotor in dreidimensionaler Ansicht mit Nabe,
- Fig. 4: einen Rotor ohne Nabe in dreidimensionaler Ansicht,
- Fig. 5: einen Teil eines Schaufelblattes in teilweise aufgebrochener Darstellung mit wabenförmigen Hohlräumen,
- Fig. 6: einen Schnitt durch ein poröses Material,
- Fig. 7: dreidimensional eine mögliche Form von Hohlräumen,
- Fig. 8: dreidimensional eine weitere mögliche Form von Hohlräumen,
- Fig. 9: eine Struktur mit kreiszylindrischen Hohlräumen,
- Fig. 10: eine Struktur mit sechseckigen wabenförmigen Hohlräumen in dichtester Anordnung und
- Fig. 11: eine Struktur mit achteckigen Wabenräumen und dazwischen liegenden weiteren Hohlräumen.

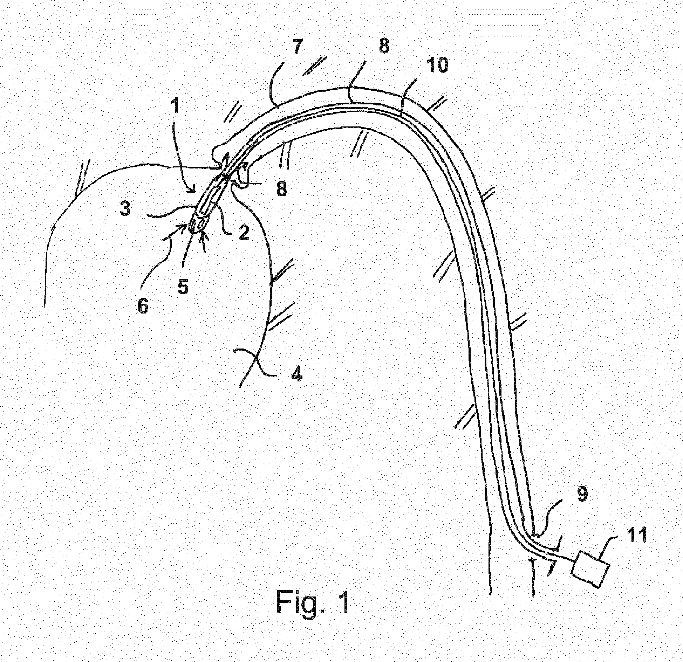

Fig. 1 zeigt eine Axialpumpe 1 mit einem Rotor 2 und einem Gehäuse 3 im Inneren einer Herzkammer 4 in schematischer Ansicht.

Innerhalb der Herzkammer 4 wird von der Pumpe 1 durch Öffnungen 5 Blut angesaugt, wie durch die Pfeile 6 angedeutet. Das Blut wird innerhalb eines Blutgefäßes 7 in Richtung der Pfeile 8 wieder ausgestoßen, und damit wird die Pumpfunktion des Herzens ersetzt oder unterstützt.

Die Pumpe 1 ist am distalen Ende eines Hohlkatheters 8 angeordnet, der durch das Blutgefäß 7 hindurch in die Herzkammer 4 eingeschoben ist und dessen proximales Ende durch eine Schleuse 9 aus dem Blutgefäß und letztlich aus dem Körper des Patienten herausragt.

Innerhalb des Hohlkatheters 8 ist eine Antriebswelle 10 vorgesehen, die mittels eines außerhalb des Körpers angeordneten Motors 11 mit hoher Drehzahl, typisch oberhalb von 10.000 Umdrehungen pro Minute, antreibbar ist. In der Pumpe 1 ist der Rotor 2 an die Welle 10 angeschlossen und rotiert mit dieser.

Die Pumpe 1 weist im Betrieb innerhalb der Herzkammer 4 einen größeren Durchmesser auf als während des Einführens durch das Blutgefäß 7. Sie kann insbesondere einen größeren Durchmesser aufweisen als den Innendurchmesser des Blutgefäßes.

Zum Entfernen der Pumpe aus dem Körper wird diese wieder komprimiert und durch die Schleuse 9 zurückgezogen.

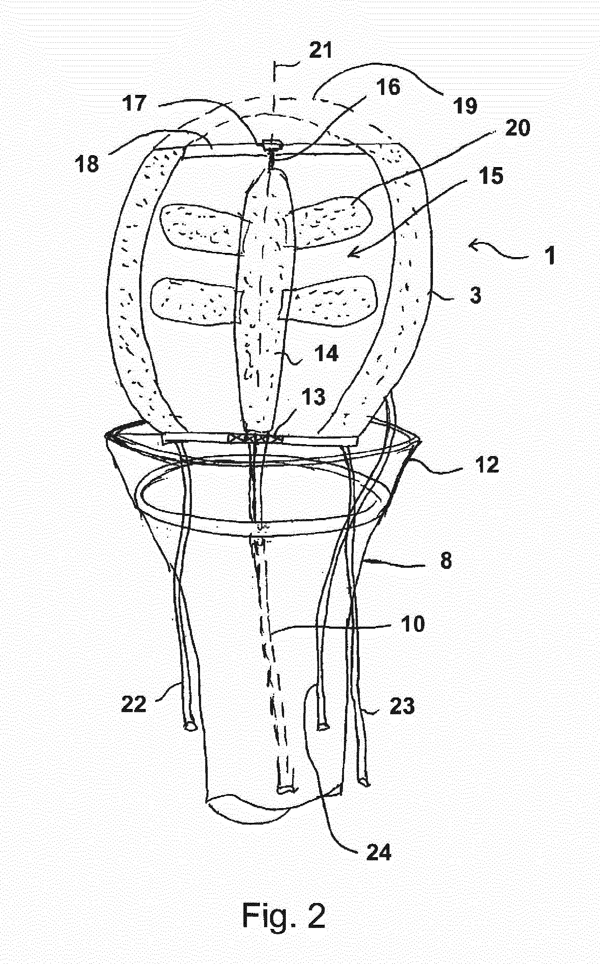

Die Fig. 2 zeigt schematisch in vergrößerter Form die Pumpe, wobei das Ende des Hohlkatheters 8 im unteren Bereich mit einem Kompressionstrichter 12 dargestellt ist.

Durch den Hohlkatheter 8 hindurch verläuft die Welle 10, und diese ist in einem Lager 13 am proximalen Ende des Pumpengehäuses 3 drehbar gelagert. Die Lagerung kann so ausgeführt sein, dass sie zugfest ausgestaltet ist, so dass an der Welle 10 das Pumpengehäuse 3 in den Kompressionstrichter 12 zumindest ein Stück weit zurückziehbar und damit gleichzeitig radial komprimierbar ist. Das Gehäuse kann auch mittels eines zusätzlichen, durch den Hohlkatheter verlaufenden Zuges, zurückziehbar sein.

Die Welle 10 ist mit dem Nabenkörper 14 des Rotors 15 verbunden, der seinerseits entweder direkt oder über einen Wellenfortsatz 16 am distalen Ende des Pumpengehäuses 3 abermals drehbar in einem zweiten Lager 17 gelagert ist. Auch dieses Lager kann zugfest ausgeführt sein, um Zugkräfte mittels der Welle 10 und des Rotors 15 auf das Gehäuse zu übertragen.

Das Lager 17 ist in einer Strebenanordnung 18 des Pumpengehäuses 3 befestigt, die genug Öffnungen aufweist, um Blut oder andere Körperflüssigkeiten zum Rotor einströmen zu lassen.

Mit 19 ist die vordere Kontur des Pumpengehäuses bezeichnet, die gitterförmig ausgestaltet ist, um einerseits beim Anstoßen der Pumpe an Körpergewebe den direkten Kontakt zum Rotor zu vermeiden und andererseits beim Ansaugen größere Partikel fernzuhalten.

Beim Einbringen der Pumpe kann zunächst das Pumpengehäuse 3 und der Rotor 15 stark radial komprimiert sein und am distalen Ende des Hohlkatheters 8 in diesem gelagert sein. Nach dem Einbringen in eine Herzkammer kann dann mittels der Welle die Pumpe ein Stück weit aus dem Katheter 8 herausgeschoben werden und sich selbsttätig aufgrund elastischer Effekte entfalten. Das Pumpengehäuse 3 entfaltet sich dabei auf den dargestellten Durchmesser, und gleichzeitig richten sich die Schaufelblätter 20 vom Nabenkörper 14 weg auf und entfernen sich von der Rotationsachse 21.

Das Herausschieben der Pumpe 1 aus dem Hohlkatheter 8 kann alternativ oder zusätzlich zu der Schubbewegung über die Welle 10 auch durch weitere Züge 22, 23 realisiert werden, die eng im oder an dem Hohlkatheter geführt sind und damit sowohl Zug- als auch Druckbewegungen zulassen. Diese Züge 22, 23 können am proximalen Ende außerhalb des Patientenkörpers an einem Manipulationsring befestigt werden, der von außen geschoben und gezogen werden kann. Die Züge können beispielsweise in Führungen an der Außenseite des Hohlkatheters eng und axial verschiebbar geführt sein.

Das Pumpengehäuse 3 kann aus einem offenporigen Schaumstoff oder einem geschlossenporigen Schaumstoff bestehen und hierdurch elastisch ausgeführt sein. Es können dort jedoch auch größere Hohlräume vorgesehen sein, die beispielsweise mittels eines Schlauches 24, der am proximalen Ende mit einem Gasreservoir oder einer Pumpe verbunden ist, abgesaugt oder mit einem Fluid angefüllt werden, um die Pumpe zu komprimieren oder zu expandieren/dekomprimieren.

Mit der Kompressionsbewegung des Gehäuses kann auch der Rotor 15 durch radial auf ihn ausgeübten Druck komprimiert werden. Der Rotor kann jedoch auch selbständig ebenfalls durch Absaugen eines Fluids aus entsprechenden Hohlräumen komprimiert werden, oder seine Kompression kann durch einen derartigen Effekt zumindest unterstützt werden.

Ein entsprechender Kompressions- und Dekompressionseffekt kann jedoch auch allein durch das Herausschieben der Pumpe aus dem Hohlkatheter und das Einziehen in den Kompressionsstutzen 12 bewerkstelligt werden.

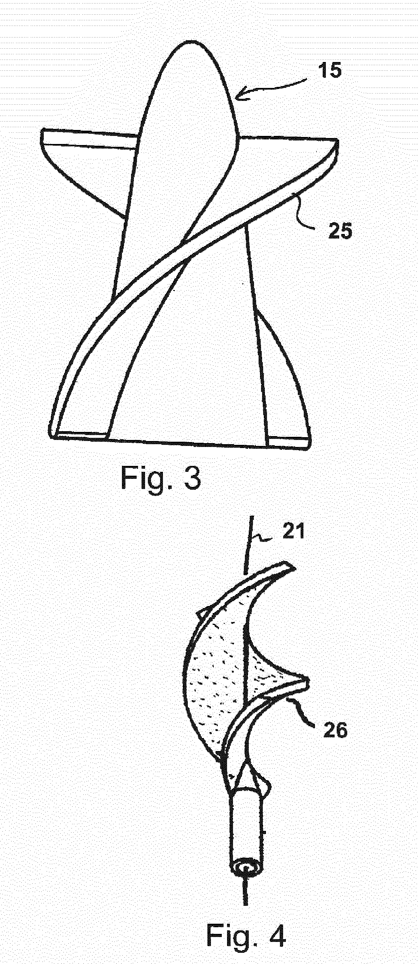

In der Fig. 3 ist in dreidimensionaler Ansicht ein Rotor mit einem umlaufenden Schaufelblatt 25 gezeigt, wobei Rotor und Schaufelblatt einstückig beispielsweise aus einem Schaumstoffmaterial, z. B. aus Polyurethan, hergestellt sein können. Es können alternativ oder zusätzlich dazu auch größere Hohlräume, insbesondere in der Nabe, aber auch im Schaufelblatt 25, vorgesehen sein.

Fig. 4 zeigt ein Schaufelblatt 26, das selbsttragend ist, beispielsweise aus einem Schaumstoff bestehen kann und nabenlos ausgeführt ist. Es ist beispielsweise aus einem flachen Material ausgeschnitten und am proximalen und distalen Ende gegeneinander um eine Längsachse 21 verdreht, um die entsprechende Wendelform zu erzeugen. Beispielsweise kann ein derartiges Schaufelblatt aus einem Schaumstoff bestehen, aus einem flachen Schaumstoffmaterial entsprechend ausgeschnitten werden, danach in die Wendelform gebracht und darauf folgend erhitzt werden, um nach dem Abkühlen die Wendelform zu stabilisieren. Danach ist der Körper stabil genug, um im Pumpbetrieb die gewünschte Form aufrechtzuerhalten, kann jedoch dennoch bei Anwendung einer entsprechenden Kompressionskraft radial komprimiert werden.

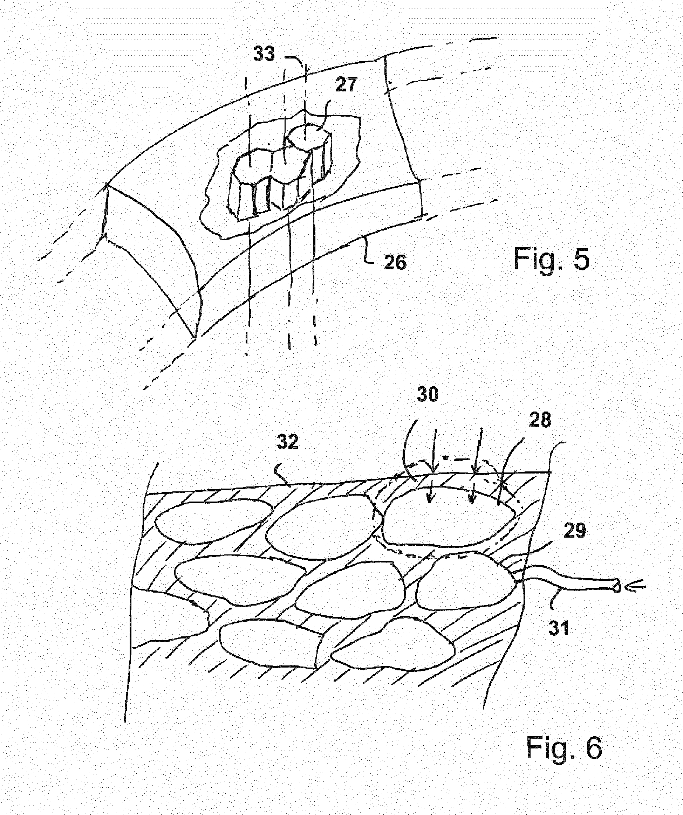

In der Fig. 5 ist schematisch ein Ausschnitt aus einem Schaufelblatt 26 gezeigt, wobei dargestellt ist, dass wabenförmige Hohlräume 27, die im Querschnitt sechseckig ausgebildet sind, mit ihren Längsachsen 33 senkrecht zur Schaufelblattfläche stehen. Auf diese Weise kann eine stark anisotrope Stabilität realisiert werden, die dazu führt, dass das Schaufelblatt in Richtung senkrecht zu seiner Förderfläche und in Umfangsrichtung auf ein Fluid große Kräfte ausüben kann, ohne sich nennenswert zu verformen, dass das Schaufelblatt jedoch durch Wirkung von radialen Kräften in Bezug auf seine Rotationsachse leichter komprimierbar ist.

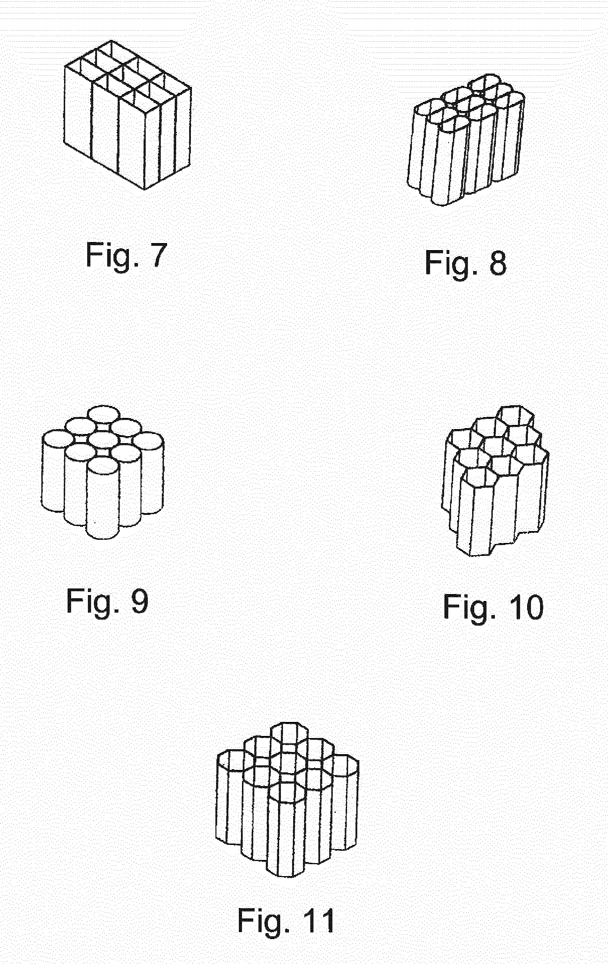

Anstelle der wabenförmigen Hohlräume 27 sind auch im Querschnitt anders geformte Hohlräume denkbar, wie diese in den Fign. 7-11 dargestellt sind. Dabei zeigt Fig. 7 quaderförmige Hohlräume, Fig. 8 Hohlräume in Strangform mit abgerundeter Quaderform, Fig. 9 Kreiszylinder, Fig. 10 sechseckige Waben in dichtester Packung und Fig. 11 achteckige Waben in einer aufgelockerten Anordnung mit im Querschnitt viereckigen Zwischenräumen.

Innerhalb eines etwa vorhandenen Nabenkörpers können derartige Hohlräume beispielsweise mit ihren Längsachsen in Umfangsrichtung gegenüber der Rotationsachse 21 der Nabe ausgerichtet sein.

Fig. 6 zeigt in stark vergrößerter, mikroskopischer Darstellung einen Schaumstoff 32 mit geschlossenen Poren 28, 29, wobei in einer Variante (Hohlraum 28) das Material der Wände zwischen den Poren als semipermeable Membran ausgebildet ist.

Eine solche Membran lässt die Diffusion bestimmter Flüssigkeiten zu, was beispielsweise für einen osmotischen Effekt genutzt werden kann. Werden die Hohlräume/Poren 28 beispielsweise mit einer Flüssigkeit gefüllt, in der hochkonzentriert ein Salz gelöst ist, und wird der Schaumstoff in eine Flüssigkeit gebracht, die eine geringere Lösungskonzentration aufweist, so tendiert die Konstellation dazu, die Konzentrationen beider Flüssigkeiten dadurch aneinander anzunähern, dass das Lösungsmittel von außen in das Innere das Hohlraums 28 durch die Membran 30 eindiffundiert. Hierdurch wird ein erhöhter osmotischer Druck erzeugt, der zum Aufpumpen des Hohlraums 28 bis in die gestrichelt dargestellte Form genutzt werden kann. Hierdurch kann eine Expansion und Versteifung des Schaumstoffs realisiert werden.

Dieser Effekt kann auch für größere Hohlräume im Rotorkörper gezielt genutzt werden. Alternativ können auch Quellvorgänge zur Expansion des Rotors genutzt werden.

Im Zusammenhang mit dem Hohlraum 29 ist ein Schlauch 31 dargestellt, der symbolisiert, dass entsprechende Hohlräume auch über einzelne oder kollektive Zuleitungen mit einem Fluid gefüllt werden können oder dass ein solches Fluid aus ihnen abgesaugt werden kann, um entsprechende Dekompressions-/Kompressionsvorgänge zu steuern.

Die Erfindung schafft somit einen Rotor, der in hohem Maße komprimierbar ist, wobei zu seiner Herstellung weitgehend anderweitig bereits übliche Materialien verwendet werden können, die größtenteils auch im medizinischen Bereich schon erprobt sind. Trotz eines hohen möglichen Kompressionsgrades wird damit ein zuverlässiges Funktionieren einer entsprechenden Fluidpumpe gewährleistet.

Die Erfindung wird durch die Ansprüche definiert. Die nachfolgenden Aspekte sind nützlich zum Verständnis der Erfindung und bilden einen Teil der Offenbarung: Ein Aspekt betrifft einen komprimierbaren Rotor (2,15) für eine Fluidpumpe (1) mit wenigstens einem Schaufelblatt (20,26) und wenigstens einem verformbaren, mit einem Fluid gefüllten oder füllbaren Hohlraum (27, 28, 29) . Gemäß einem weiteren Aspekt ist/sind bei dem Rotor der/die Hohlraum/Hohlräume (27,28,29) geschlossen. Gemäß einem weiteren Aspekt ist/sind bei dem Rotor der/die Hohlraum/Hohlräume (27,28,29) wenigstens teilweise von einer teildurchlässigen Membran begrenzt. Gemäß einem weiteren Aspekt ist bei dem Rotor der wenigstens eine Hohlraum (28) mit einer Flüssigkeit gefüllt, die im Zusammenwirken mit der Membran und einer Flüssigkeit, in der die Pumpe einsetzbar ist, insbesondere Blut, eine osmotische Diffusion in den Hohlraum (28) hinein mit entsprechender Druckerhöhung bewirkt. Gemäß einem weiteren Aspekt ist bei dem Rotor wenigstens ein Teil der Hohlräume (27,28,29) von festem Material(32) des Rotors umgeben und über Öffnungen mit dem Außenbereich und/oder untereinander verbunden. Gemäß einem weiteren Aspekt besteht der Rotor (2,15) wenigstens teilweise aus einem porösen Material(32), insbesondere Schaumstoff. Gemäß einem weiteren Aspekt weist der Rotor wenigstens einen Hohlraum (27) auf, der in einer ersten Richtung (33) eine größere Ausdehnung aufweist als in den im Wesentlichen senkrecht dazu stehenden Richtungen. Gemäß einem weiteren Aspekt ist/sind bei dem Rotor der/die Hohlraum/Hohlräume (27) im Querschnitt rund oder vieleckig, insbesondere achteckig, sechseckig, dreieckig oder viereckig ausgebildet. Gemäß einem weiteren Aspekt weisen der/die Hohlraum/Hohlräume (27) Strangform auf. Gemäß einem weiteren Aspekt sind die Hohlräume (27) mit der Richtung ihrer größten Stabilität, insbesondere ihrer Längsachse (32), in Richtung der im Betrieb auftretenden Druckkräfte innerhalb des Rotors ausgerichtet. Gemäß einem weiteren Aspekt ist/sind der/die Hohlraum/ Hohlräume (27,28,29) in wenigstens einem Schaufelblatt (25,26) vorgesehen. Gemäß einem weiteren Aspekt ist das Schaufelblatt (26) selbsttragend und nabenlos ausgebildet. Gemäß einem weiteren Aspekt sind der/die Hohlraum/Hohlräume (27,28,29) in einem Nabenkörper vorgesehen. Ein weiterer Aspekt betrifft eine Fluidpumpe mit einem wie oben beschriebenen Rotor, wobei ein komprimierbares, den Rotor (2,15) umgebendes Gehäuse (3) vorgesehen ist. Gemäß einem Aspekt besteht das Gehäuse (3) wenigstens teilweise aus einem Hohlräume (27,28,29) enthaltenden Material, insbesondere einem Schaumstoff. Ein weiterer Aspekt betrifft einen komprimierbaren Rotor für eine Fluidpumpe mit wenigstens einem Schaufelblatt, wobei der Rotor so aufgebaut ist, dass er einen komprimierten und einen dekomprimierten Zustand einnehmen kann und die mittlere Dichteänderung des Rotormaterials zwischen komprimiertem und dekomprimiertem Zustand mindestens 10 % beträgt.

## Patentansprüche

1. Komprimierbarer Rotor für eine Fluidpumpe mit wenigstens einem Schaufelblatt, **gekennzeichnet durch** wenigstens einen Hohlraum (27), der in einer ersten Richtung (33) eine größere Ausdehnung aufweist als in den im Wesentlichen senkrecht dazu stehenden Richtungen, wobei der Hohlraum ein verformbarer, mit einem Fluid gefüllter oder füllbarer Hohlraum ist.

2. Rotor nach Anspruch 1, **dadurch gekennzeichnet, dass** der/die Hohlraum/Hohlräume (27) im Querschnitt rund oder vieleckig, insbesondere achteckig, sechseckig, dreieckig oder viereckig ausgebildet ist/sind.

3. Rotor nach Anspruch 1 oder 2, **dadurch gekennzeichnet, dass** der/die Hohlraum/Hohlräume (27) Strangform aufweisen.

4. Rotor nach einem der Ansprüche 1 bis 3,
**dadurch gekennzeichnet, dass** die Hohlräume (27) mit der Richtung ihrer größten Stabilität in Richtung der im Betrieb auftretenden Drucckräfte innerhalb des Rotors ausgerichtet sind.

5. Rotor nach Anspruch 4, **dadurch gekennzeichnet, dass** die Hohlräume mit der Richtung ihrer Längsachse in Richtung der im Betrieb auftretenden Druckkräfte innerhalb des Rotors ausgerichtet sind.

6. Rotor nach einem der Ansprüche 1 bis 5,
**dadurch gekennzeichnet, dass** der/die Hohlraum/ Hohlräume (27,28,29) in wenigstens einem Schaufelblatt (25,26) vorgesehen ist/sind.

7. Rotor nach einem der Ansprüche 1 bis 6,
**dadurch gekennzeichnet, dass** das Schaufelblatt (26) selbsttragend und nabenlos ausgebildet ist.

8. Rotor nach einem der Ansprüche 1 bis 7,
**dadurch gekennzeichnet, dass** der/die Hohlraum/Hohlräume (27,28,29) in einem Nabenkörper vorgesehen sind.

9. Rotor nach einem der Ansprüche 1 bis 8,
**dadurch gekennzeichnet, dass** der/die Hohlraum/Hohlräume geschlossen ist/sind.

10. Fluidpumpe mit einem Rotor nach einem der Ansprüche 1 bis 9, **dadurch gekennzeichnet, dass** ein komprimierbares, den Rotor (2,15) umgebendes Gehäuse (3) vorgesehen ist.

11. Fluidpumpe nach Anspruch 10, **dadurch gekennzeichnet, dass** das Gehäuse (3) wenigstens teilweise aus einem Hohlräume (27,28,29) enthaltenden Material, insbesondere einem Schaumstoff, besteht.

## Claims

1. Compressible rotor for a fluid pump having at least one blade, **characterised by** at least one cavity (27) which has a greater extension in a first direction (33) than in the directions essentially perpendicular thereto, wherein the cavity is a deformable cavity that is or can be filled with fluid.

2. Rotor according to claim 1, **characterised in that** the cavity/cavities (27) is/are configured, in cross-section, to be round or polygonal, in particular octagonal, hexagonal, triangular or square.

3. Rotor according to claim 1 or 2, **characterised in that** the cavity/cavities (27) have a strand shape.

4. Rotor according to any one of the claims 1 to 3, **characterised in that** the cavities (27) are orientated with the direction of their greatest stability in a direction of the pressure forces which arise within the rotor during operation.

5. Rotor according to claim 4, **characterised in that** the cavities are orientated with the direction of their longitudinal axis, in a direction of the pressure forces which arise within the rotor during operation.

6. Rotor according to any one of the claims 1 bis 5, **characterised in that** the cavity/cavities (27, 28, 29) is/are provided in at least one blade (25, 26).

7. Rotor according to any one of the claims 1 bis 6, **characterized in that** the blade (26) is configured to be self-supporting and hub-free.

8. Rotor according to any one of the claims 1 bis 7, **characterised in that** the cavity/cavities (27, 28, 29) are provided in a hub body.

9. Rotor according to any one of the claims 1 bis 8, **characterised in that** the cavity/cavities is/are closed.

10. Fluid pump having a rotor according to one of the claims 1 to 9, **characterised in that** a compressible housing (3) surrounding the rotor (2, 15) is provided.

11. Fluid pump according to claim 10, **characterised in that** the housing (3) consists at least partially of a material comprising cavities (27, 28, 29), in particular a foam.

## Revendications

1. Rotor comprimable pour une pompe à fluide, avec au moins une pale, **caractérisé par** au moins une cavité (27) qui présente, dans une première direction (33), une extension supérieure à celle dans les directions globalement perpendiculaires à celle-ci, dans lequel la cavité est une cavité déformable qui est ou peut être remplie avec un liquide.

2. Rotor selon la revendication 1, **caractérisé en ce que** la ou les cavité(s) (27) présente(nt) une section transversale de forme ronde ou polygonale, plus particulièrement octogonale, hexagonale, triangulaire ou quadrangulaire.

3. Rotor selon la revendication 1 ou 2, **caractérisé en ce que** la ou les cavité(s) (27) présente(nt) une forme de toron.

4. Rotor selon l'une des revendications 1 à 3, **caractérisé en ce que** les cavités (27) sont orientées avec leur direction de plus grande stabilité, en direction des forces de pression survenant lors du fonctionnement à l'intérieur du rotor.

5. Rotor selon la revendication 4, **caractérisé en ce que** les cavités sont orientées avec leur direction de leur axe longitudinal, en direction des forces de pression survenant lors du fonctionnement à l'intérieur du rotor.

6. Rotor selon l'une des revendications 1 à 5, **caractérisé en ce que** la ou les cavité(s) (27, 28, 29) est/sont prévue(s) dans au moins une pale (25, 26).

7. Rotor selon l'une des revendications 1 à 6, **caractérisé en ce que** la pale (26) est conçue de manière autoportante ou sans moyeu.

8. Rotor selon l'une des revendications 1 à 7, **caractérisé en ce que** la ou les cavité(s) (27, 28, 29) est/sont prévue(s) dans un corps de moyeu.

9. Rotor selon l'une des revendications 1 à 8, **caractérisé en ce que** la ou les cavité(s) est/sont fermée(s).

10. Pompe à fluide avec un rotor selon l'une des revendications 1 à 9, **caractérisée en ce qu'**un carter (3) comprimable entourant le rotor (2, 15) est prévu.

11. Pompe à fluide selon la revendication 10, **caractérisé en ce que** le carter (3) est constitué au moins partiellement d'un matériau contenant des cavités (27, 28, 29), plus particulièrement un matériau alvéolaire.
